# EUROPEAN PATENT APPLICATION

(11) **EP 4 595 945 A2**
(43) Date of publication of application: **06.08.2025**
(21) Application number: 25183297.8
(22) Date of filing: 03.11.2020
(51) Int. Cl.: A61K 8/49

(54) **SUNSCREEN COMPOSITION WITH LOW STICKINESS**

(30) Priority: 05.11.2019 EP 19207108
(62) Divisional of application: 20803453.8
(71) Applicant: DSM IP Assets B.V., 6221 BE Maastricht (NL)
(72) Inventor: JANSSEN, Anne, 4303 Kaiseraugst (CH); RADOMSKY, Karina, 4303 Kaiseraugst (CH); RUDOLPH, Thomas, 4303 Kaiseraugst (CH)
(74) Representative: dsm-firmenich IP

(57) **Abstract**

The present invention relates to a topical preparation comprising a UV filter combination of certain microfine zinc oxide, 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 2-phenylbenzimidazole-5-sulfonic acid and/or one or more salts thereof and 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone)

## Description

The present invention relates to a topical preparation comprising a UV filter combination of microfine zinc oxide, 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 2-phenylbenzimidazole-5-sulfonic acid and/or one or more salts thereof and 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone).

It is well known that the ultraviolet (UV) radiation in sun-light has a damaging effect on the skin. Besides the well-known acute damage (sunburn), there is also long-term damage such as an increased risk of developing skin cancer. Moreover, exposure to UV-radiation is responsible for most of the wrinkles and age spots on our faces. Accordingly, to protect the skin against the detrimental effects of UV-light, a range of photoprotective UV-filter substances have been developed that can be incorporated into topical sunscreen preparations.

Modern sunscreens should effectively and safely protect the skin against the harmful effects of UV-radiation. Thus, sunscreens generally contain various UV-B (wavelength: 280-320 nm) and UV-A (wavelength: 320-400 nm) filters. These filters, however, often render the sunscreen sticky, which particularly in beach use means that sand ends up adhering to parts of the skin to which cream has been applied. The greater the UV filter content of a preparation, the greater this problem. To counteract the stickiness problem, consumers tend to use less sunscreen, which however leads to an insufficient protection.

Even though there has been no lack of attempts to develop sunscreen compositions exhibiting a reduced stickiness, this problem has not yet been solved to ultimate satisfaction, particularly in the case of preparations featuring a high sun protection factor.

It is therefore an ongoing need to develop novel sunscreen formulations which exhibit a reduced stickiness.

It would thus be advantageous to have available a sand-repellent sunscreen composition, more particularly a sunscreen composition having a high sun protection factor (SPF 20 or more) that exhibits particularly low sand adhesion.

In addition to the stickiness/sand adhesion, moreover, a problem of cosmetic sunscreen compositions is that very many UV filters do not have particularly good solubility in the preparations. Especially if preparations with a high sun protection factor and high UV filter content are developed, the developers face the problem of the solubility of triazine derivatives. In the past, in order to solve this problem, the liquid UV-B filter octocrylene has been used as UV filter and solvent.

The disadvantage of the prior art lies, then, in the circumstance that the use of octocrylene, in spite of its approval by the approval authorities, is not entirely uncontroversial and results, when evaluations are undertaken in certain consumer magazines (e.g., the German Oko-test), in "markdowns" in the scoring of the product. The reason given for this negative evaluation is that certain scientists suspect that this UV filter might possibly have hormonal activity. Even when no negative effects in people have become apparent in spite of the decades-long use of this UV filter worldwide in sunscreen compositions, there is still a desire among consumers to avoid preparations containing such ingredients.

It, therefore, is desirable to overcome the disadvantages of the prior art and to develop a sand-repellent sunscreen composition with a high sun protection factor, in which the organic UV filters such as the triazine derivatives are stably dissolved. Ideally it ought to be possible to achieve the object without using octocrylene as solvent and stabilizer.

Thus, the present invention provides a topical sunscreen composition. The topical composition comprises a UV filter combination of
a.) a microfine zinc oxide, wherein preferably the microfine zinc oxide has a mean particle size Dn50 as determined by Laser diffraction selected in the range from 50 to 200 nm and a surface coating selected from the group consisting of aluminum stearate, stearic acid, methicone, simethicone, triethoxycaprylylsilane and octyltrimethoxysilane,
b.) 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine),
c.) 2-phenylbenzimidazole-5-sulfonic acid and/or one or more salts thereof and
d.) 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone).

The term microfine zinc oxide refers to any zinc oxide particles suitable for use as UV-filter, i.e. a zinc oxide having a particle size which is principally useful for incorporation into a sunscreen composition. Advantageously the microfine zinc oxide consists of particles having a primary particle size of less than about 300 nm, e.g., less than about 200 nm, or less than about 150 nm. Most preferably, in all embodiments of the present invention the mean particle size of the microfine zinc oxide according to the present invention is selected in the range from 30 to 200 nm, preferably in the range from 50 to 200nm, more preferably in the range from 75 to 150 nm, most preferably in the range from 90 to 130 nm. Further suitable ranges encompass from 30 nm to 150 nm and from 30 nm to 130 nm.

The term 'mean particle size' as used herein refers to the mean number-based particle size distribution Dn50 (also known as Dn0.5) as determined by laser diffraction e.g. with a Horiba particle size distribution analyzer LA-960 or a Malvern Mastersizer 2000 (ISO 13320:2009).

The Zinc oxide according to the present invention furthermore preferably has a Dn1 (1% below this size) >20 nm.

According to the invention, it is also advantageous for zinc oxide particles to be surface-coated. The surface coating may comprise providing the metal oxide particles with a thin hydrophilic or hydrophobic inorganic or organic layer by methods known per se. According to the present invention the different surface coatings can also comprise water. As a result of the surface treatment, the metal oxide is given a hydrophilic, amphiphilic or hydrophobic character.

Examples of inorganic surface coatings which are suitable for the purposes of the instant invention comprise aluminum oxide (Al₂0₃), aluminum hydroxide Al(OH)₃, aluminum oxide hydrate (also: Alumina, CAS-No.: 1333-84-2), sodium hexametaphosphate (NaPO₃)₆, sodium meta-phosphate (NaPO₃)ₙ, silicon dioxide (SiO₂) (also: Silica, CAS-No.: 7631-86-9), and iron oxide (Fe₂O₃). These inorganic surface coatings can be present on their own, in combination and/or in combination with organic coating materials.

Examples of organic surface coatings which are suitable for use in the present invention include vegetable or animal aluminum stearate, vegetable or animal stearic acid, lauric acid, dimethylpolysiloxane (also: dimethicone), methylpolysiloxane (methicone), simethicone (a mixture of dimethylpolysiloxane with an average chain length of about 200 to about 350 dimethylsiloxane units and silica gel), triethoxycaprylylsilane, and octyltrimethoxysilane. These organic surface coatings can be present on their own, in combination and/or in combination with inorganic coating materials. Preferably, the zinc oxide according to the present invention is uncoated or coated with dimethicone, methicone or triethoxycaprylylsilane, most preferably the zinc oxide according to the present invention is surface coated with triethoxycaprylylsilane.

The amount of the coating is preferably selected in the range of 0.1 to 25 wt.-%, preferably in the range of 0.25 to 10 wt.-%, most preferably in the range of 0.5 to 7.5 wt.-% or even more preferably in the range of 1 to 5 wt.-%, based on the weight of the uncoated zinc oxide

It is furthermore preferred that the zinc oxide is a white powder consisting of zinc oxide present as wurtzite crystal structures.

Zinc oxide particles for use according to the present invention and pre-dispersions of zinc oxide particles are e.g. available from Tayca as MZ-505S (5% methicone coating), from Kobo as ZnO-C-DS4 (dimethicone coating) or from DSM Nutritional Products Ltd as PARSOL^{®} ZX (2-3.5% triethoxycaprylylsilane coating).

Particularly advantageous zinc oxide according to the present invention is obtained by dissolving Zinc oxide in water under strong acidic condition (e.g. HCl) and high temperature, followed by recrystallisation of the zinc oxide particles through neutralisation with NaOH and coating of the thus obtained particles with triethoxycaprylylsilane, followed by dehydration, drying and milling.

Most preferably, in all embodiments according to the present invent, the zinc oxide is a white powder consisting of zinc oxide present as wurtzite crystal structures, coated with triethoxycaprylylsilane, which has a zinc oxide content of 96-98%, a triethoxycaprylylsilane content of 2-3.5 % and a mean particle size of 90 to 130 nm, which is commercially available as PARSOL^{®} ZX.

In all embodiments of the present invention, the amount of the microfine zinc oxide is preferably selected in the range from 0.1 wt.-% to 20 wt.-%, more preferably in the range from 0.5 wt.-% to 15 wt.-%, most preferably in the range from 1 wt.-% to 10 wt.-%, such as in the range from 5 wt.-% to 10 wt.-%, based on the total weight of the topical composition.

In all embodiments of the present invention, the amount of the 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine) is preferably selected in the range from 0.1 wt.-% to 10 wt.-%, more preferably in the range from 0.5 wt.-% to 7.5 wt.-%, most preferably in the range from 1 wt.-% to 5 wt.-%, based on the total weight of the topical composition.

In all embodiments of the present invention, the amount of the 2-phenylbenzimidazole-5-sulfonic acid and/or one or more salts thereof is preferably selected in the range from 0.1 wt.-% to 10 wt.-%, more preferably in the range from 0.5 wt.-% to 7.5 wt.-%, most preferably in the range from 1 wt.-% to 5 wt.-%, based on the total weight of the topical composition.

In all embodiments of the present invention, the amount of 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone) is preferably selected in the range from 0.1 wt.-% to 10 wt.-%, more preferably in the range from 0.5 wt.-% to 7.5 wt.-%, most preferably in the range from 1 wt.-% to 5 wt.-%, based on the total weight of the topical composition.

The term 'topical' as used herein is understood here to mean external application to keratinous substances, which are in particular the skin, scalp, eyelashes, eyebrows, nails, mucous membranes and hair, preferably the skin of a human.

As the topical compositions according to the invention are intended for topical application, it is well understood that they comprise a physiologically acceptable medium, i.e. a medium compatible with keratinous substances, such as the skin, mucous membranes, and keratinous fibers. In particular, the physiologically acceptable medium is a cosmetically acceptable carrier.

The term 'cosmetically acceptable carrier' as used herein refers to all carriers and/or excipients and/ or diluents conventionally used in topical cosmetic compositions such as in particular in skin care preparations.

The exact amount of carrier will depend upon the actual level of the UV filters and any other optional ingredients that one of ordinary skill in the art would classify as distinct from the carrier (e.g., other active ingredients).

In an advantageous embodiment, the topical compositions according to the present invention comprise from 50% to 99%, preferably from 60% to 98%, more preferably from 70% to 98%, such as in particular from 80% to 95% of a carrier, based on the total weight of the topical composition.

In a particular advantageous embodiment, the carrier consists furthermore of at least 40 wt.-%, more preferably of at least 50 wt.-%, most preferably of at least 55 wt.-% of water, such as in particular of 55 to 90 wt.-% of water.

In particular, the topical composition according to the present invention are cosmetic or pharmaceutical compositions, preferably cosmetic (non-therapeutic) compositions.

In one embodiment, the topical compositions according to the present invention are applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

Preferred topical compositions according to the invention are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment, the topical compositions according to the invention are light-protective preparations (sun care products, sunscreens), such as sun protection milks, sun protection lotions, sun protection creams, sun protection oils, sun blocks or tropical's or day care creams with a SPF (sun protection factor). Of particular interest are sun protection creams, sun protection lotions, sun protection milks and sun protection preparations.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions.

If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The topical compositions according to the present invention may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

The topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art and illustrated in the examples.

In one advantageous embodiment, O/W emulsifier is a phosphate ester emulsifier. Among the preferred phosphate ester emulsifier are C₈₋₁₀Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C₆₋₁₀ Pareth-4 Phosphate, C₁₂₋₁₅ Pareth-2 Phosphate, C₁₂₋₁₅ Pareth-3 Phosphate, DEA-Ceteareth-2 Phosphate, DEACetyl Phosphate, DEA-Oleth-3 Phosphate, Potassium cetyl phosphate, Deceth-4 Phosphate, Deceth-6 Phosphate and Trilaureth-4 Phosphate. A particular phosphate ester emulsifier according to the invention is potassium cetyl phosphate e.g. commercially available as Amphisol^{®} K at DSM Nutritional Products Ltd Kaiseraugst.

Further suitable O/W emulsifiers according to the present invention encompass PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

Particularly advantageous O/W emulsifiers according to the present invention are one or more of Polyglyceryl-3 Methylglucose Distearate, Lauryl Glucoside (and) Polyglyceryl-2 Dipolyhydroxystearate, Glyceryl Sterate Citrate, Sodium Cetearyl Sulfate, Cetearyl Glucoside; Polyglyceryl-6 Stearate (and) Polyglyceryl-6 Behenate, Cetearyl Olivate (and) Sorbitan Olivate, Arachidyl Alcohol (and) Behenyl Alcohol (and) Arachidyl Glucosides, Cetearyl Alcohol (and) Coco-Glucoside, Coco-Glucoside (and) Coconut Alcohol, PEG-100 Stearate (and) Glyceryl Stearate, Sodium Stearoyl Glutamate, Steareth-20, Steareth-21, Steareth-25, Steareth-2, Ceteareth-25 and Ceteareth-6 (all listed by their INCI names).

Most preferred O/W emulsifiers according to the present invention are one or more of Ceteareth-6 (optionally in admixture with stearyl alcohol) and Ceteareth-25.

The at least one O/W respectively Si/W emulsifier is preferably used in an amount of 0.5 to 10 wt.-% such as in particular in the range of 0.5 to 5 wt.-% such as most in particular in the range of 0.5 to 4 wt.-%, based on the total weight of the composition.

Suitable W/O- or W/Si-emulsifiers according to the present invention are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The topical compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 7 wt.-%, such as most in particular in the range of 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), glyceryl stearate, glyceryl myristate (Estol 3650), hydrogenated coco-glycerides (Lipocire Na10) as well as mixtures thereof.

In a particular advantageous embodiment according to the present invention the emulsifier is selected from the group of Ceteareth-6 and/ or Ceteareth-25 and the co-surfactant is selected from the group of behenyl alcohol, cetyl alcohol, cetearyl alcohol and/ or stearyl alcohol.

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

According to an advantageous embodiment of the invention the compositions constitute cosmetic composition and are intended for topical application to the skin.

Finally, a subject-matter of the invention is a method for the cosmetic treatment of keratinous substances such as in particular the skin, wherein a composition as defined above is applied to the said keratinous substances such as in particular to the skin. The method is in particular suitable to protect the skin against the adverse effects of UV-radiation such as in particular sun-burn and/ or photoageing.

In one advantageous aspect of the invention, the topical compositions according to the present invention do not contain (i.e. are free of) 3-(4-methylbenzylidene)-camphor, 2-hydroxy-4-methoxybenzophenone (INCI: Oxybenzone), and ethylhexyl 2-cyano-3,3-diphenylacrylate (INCI: Octocrylene).

In another advantageous embodiment, the topical compositions according to the present invention do not contain (i.e. are free of) ethylhexyl triazone.

In another advantageous aspect of the invention, the topical compositions according to the present invention further comprise one or more of dibutyl adipate, dicaprylyl carbonate, C12-C15 alkylbenzoate, Caprylyl Carbonate, Capric/Caprylic Triglyceride as well as mixtures thereof, preferably C12-C15 alkylbenzoate and/ or dicaprylyl carbonate.

In a still further advantageous aspect of the invention, the topical compositions of the present invention further comprise a preservative and/ or a preservative booster, preferably selected from the group consisting of phenoxyethanol, ethylhexylglycerin, hexylglycerin, glyceryl caprylate, caprylyl glycol, 1,2-hexanediol, propanediol, propylene glycol, p-hydroxyacetophenone as well as mixtures thereof, most preferably selected from the group of p-hydroxyacetophenone, phenoxyethanol and hexylglycerine as well as mixtures thereof. When present, the preservative respectively the preservative booster is preferably used in an amount of 0.01 to 2 wt.-%, more preferably in an amount of 0.05 to 1.5 wt.-%, most preferably in an amount of 0.1 to 1.0 wt.-%, based on the total weight of the composition.

In another advantageous aspect, the topical compositions according to the present invention are free of any parabenes, benzethoniumchlorid, piroctone olamine, lauroylarginat, methylisothiazolinon, chlormethylisothiazolinon, bronopol, benzalkoniumchloride, formaldeh releasing compounds, salicylic acid, triclosan, DMDM hydantoin, chlorphenesin and IPBC (lodopropinylbutyl carbamate).

In a still further advantageous aspect, the topical compositions of the present invention further comprise one or more of glycerol or polyhydroxystearic acid as well as mixtures thereof.

In yet a still further advantageous aspect, the topical compositions of the present invention further comprise a thickening agent, preferably a gum such as xanthan gum or Caesalpina spinosa gum or a polyacrylate such as polyacrylate crosspolymer-6 as well as mixtures thereof.

The topical compositions according to the invention in general have a pH in the range of 3 to 10, preferably a pH in the range of 4 to 8 and most preferably a pH in the range of 4 to 7. The pH can easily be adjusted as desired with suitable acids such as e.g. citric acid or bases such as NaOH according to standard methods in the art.

The topical compositions according to the invention may further contain one or more emollients which soothe and soften the skin. As an example, the emollient may be dicaprylyl carbonate or C₁₂₋₁₅alkyl benzoate. Further emollients are silicone (dimethicone, cyclomethicone), vegetable oils (grape seed, sesame seed, jojoba, etc.), butters (cocoa butter, shea butter), alcohols (stearyl alcohol, cetyl alcohol), and petrolatum derivatives (petroleum jelly, mineral oil).

In yet another aspect, the topical composition of the invention may comprise potassium cetylphosphate as emulsifier and/or one or more of dibutyl adipate, dicaprylyl carbonate and C12-C15 alkylbenzoate.

In another aspect, the topical composition of the invention may comprise one or more fragrances selected from limonene, citral, linalool, alpha-isomethylionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic diester, cinnamal, amyl salicylate, alpha-amylcinnamaldehyde, alpha-methylionone, butylphenylmethylpropional, cinnamal, amylcinnamyl alcohol, anise alcohol, benzoin, benzyl alcohol, benzyl benzoate, benzyl cinnamate, benzyl salicylate, bergamot oil, bitter orange oil, butylphenylmethylpropional, cardamom oil, cedrol, cinnamal, cinnamyl alcohol, citronellyl methylcrotonate, citrus oil, coumarin, diethyl succinate, ethyllinalool, eugenol, Evernia furfuracea extract, Evernia prunastri extract, farnesol, guaiacwood oil, hexylcinnamal, hexyl salicylate, hydroxycitronellal, lavender oil, lemon oil, linalyl acetate, mandarin oil, menthyl PCA, methyl heptenone, nutmeg oil, rosemary oil, sweet orange oil, terpineol, tonkabean oil, triethyl citrate, vanillin.

In another aspect, the topical composition of the invention may contain at least one salt of 2-phenylbenzimidazole-5-sulfonic acid.

In another aspect, the composition may have an SPF of at least 20, preferably of at least 30.

The topical compositions of the invention manage with a surprisingly small total amount of UV filters.

In addition to the UV filter combination claimed, the preparation of the invention may comprise one or more further UV filters. These may be selected, advantageously in accordance with the invention, from phenylene-1,4-bis(2-benzimidazyl)-3,3',5,5'-tetrasulfonic acid salts; 1,4-di(2-oxo-10-sulfo-3-bornylidenemethyl)benzene and its salts; 4-(2-oxo-3-bornylidenemethyl)-benzenesulfonic acid salts; 2-methyl-5-(2-oxo-3-bornylidenemethyl)sulfonic acid salts; 2,2'-methylene-bis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-benzylidenecamphor; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; isoamyl 4-methoxycinnamoate; hexyl 2-(4'-diethylamino-2'-hydroxybenzoyl)benzoate, ethylhexyl salicylate, homomenthyl salicylate, 2-ethylhexyl 2-hydroxybenzoate; dimethicodiethyl benzalmalonate; 3-(4-(2,2-bis-ethoxycarbonylvinyl)-phenoxy)propenyl)methoxysiloxane/dimethylsiloxane copolymer; 4-(tert.-Butyl)-4'-methoxydibenzoylmethan, 1,4-di(benzoxazol-2'-yl)benzene and 2,4,6-tribiphenyl-4-yl-1,3,5-triazine.

In a preferred embodiment, the invention is free of 1,4-di(benzoxazol-2'-yl)benzene, in particular micronized 1,4-di(benzoxazol-2'-yl)benzene.

It is advantageous in accordance with the invention if the topical composition is present in the form of an emulsion or dispersion, preferably in the form of an emulsion, and more preferably in the form of an O/W emulsion.

Where the topical composition of the invention is in the form of an O/W emulsion, the preparation advantageously comprises one or more O/W emulsifiers selected from glyceryl stearate citrate, glyceryl stearate (self-emulsifying), stearic acid, stearate salts, polyglyceryl-3-methylglycose distearate, sodium cetearylsulfate, potassium cetyl phosphate, polyglyceryl-10 stearate, and sodium stearylglutamate.

Advantageously in accordance with the invention, these O/W emulsifiers of the invention may be present in the preparation in a concentration of 0.001 to 10 wt % and preferably in a concentration of 0.1 to 7 wt %, based on the total weight of the preparation.

It is preferred in accordance with the invention if the preparation comprises potassium cetyl phosphate as emulsifier.

It is further advantageous in accordance with the invention if the preparation comprises cetyl alcohol, stearyl alcohol and/or glycerylstearate.

It is of advantage in accordance with the invention if the preparation of the invention is free from polyethylene glycol, polyethylene glycol ethers, and polyethylene glycol esters (so-called PEG derivatives).

The preparation of the invention may advantageously comprise moisturizers. Moisturizers are compounds or mixtures of compounds which give cosmetic preparations the quality, after application to or distribution on the skin surface, of reducing the loss of moisture of the stratum corneum (also called transepidermal water loss (TEWL)) and/or of positively influencing the hydration of the stratum corneum.

Non-limiting examples of advantageous moisturizers for use in the present invention include glycerol, lactic acid and/or lactates, especially sodium lactate, butylene glycol, propylene glycol, biosaccharide gum-1, Glycine soya, ethylhexyloxyglycerol, pyrrolidonecarboxylic acid, and urea. Of further advantage, in particular, is the use of polymeric moisturizers from the group of the polysaccharides which are water-soluble and/or swellable in water and/or gellable with the aid of water. Especially advantageous, for example, are hyaluronic acid, chitosan and/or a fucose-rich polysaccharide which is registered in Chemical Abstracts under the registry number 178463-23-5 and is available, for example, under the Fucogel^{®}1000 name from the company SOLABIA S.A. Moisturizers may also be used advantageously as active antiwrinkle ingredients for protection from changes to the skin of the kind occurring in skin aging, for example.

The cosmetic preparations of the invention may further comprise advantageously, although not mandatorily, fillers which have the effect, for example, of further improving the sensorial and cosmetic properties of the formulations and evoking or intensifying a velvety or silky skin sensation, for example. Advantageous fillers in the sense of the present invention are starch and starch derivatives (such as tapioca starch, distarch phosphate, aluminum or sodium starch octenylsuccinate, and the like, for example), pigments which have neither primarily UV filter effect nor coloring effect (such as boron nitride, etc., for example) and/or Aerosils^{®} (CAS No. 7631-86-9) and/or talc and/or polyethylene, nylon, and silica dimethyl silylate.

Advantageous embodiments of the preparation of the present invention also include those wherein the preparation comprises one or more oils selected from butylene glycol dicaprylate/dicaprate, phenethyl benzoate, C12-15 alkyl benzoate, dibutyl adipate; diisopropyl sebacates, dicaprylyl carbonate, di-C12-13 alkyl tartrates, butyloctyl salicylates, diethylhexyl syringylidene malonates, hydrogenated castor oil dimerates, triheptanoin, C12-13 alkyl lactates, C16-17 alkyl benzoates, propylheptyl caprylates, caprylic/capric triglycerides, diethylhexyl 2,6-naphthalates, octyldodecanol, ethylhexyl cocoates.

It is preferred in accordance with the invention if the preparation comprises dibutyl adipate, dicaprylyl carbonate and/or C12-C15 alkyl benzoate.

The water phase of the preparations of the invention may advantageously comprise customary cosmetic auxiliaries, such as, for example, alcohols, particularly those of low C number, preferably ethanol and/or isopropanol, or polyols of low C number, and also ethers thereof, preferably propylene glycol, glycerol, electrolytes, self-tanning agents, and also, in particular, one or more thickeners, which may be advantageously selected from the group of silicon dioxide, aluminum silicates, polysaccharides and/or derivatives thereof, e.g., hyaluronic acid, xanthan gum, hydroxypropylmethylcellulose, particularly advantageously from the group of polyacrylates, preferably a polyacrylate from the group referred to as Carbopols, examples being carbopols of types 980, 981, 1382, 2984, and 5984, in each case individually or in combination. Further thickeners advantageous in accordance with the invention are those having the INCI designation Acrylates/C10-30 Alkyl Acrylate Crosspolymer (e.g., Pemulen TR 1, Pemulen TR 2, Carbopol 1328 from NOVEON) and also Aristoflex AVC (INCI: Ammonium Acryloyldimethyltaurate/VP Copolymer) as well as Simugel NS (INCI: Hydroxyethyl Acrylate/Sodium Acryloyldimethyl Taurate Copolymer & Squalane & Polysorbate 60).

It is preferred here in accordance with the invention if the preparation comprises xanthan gum, crosslinked acrylate/C10-C30 alkyl acrylate polymer, Hydroxyethyl Acrylate/Sodium Acryloyldimethyl Taurate Copolymer & Squalane & Polysorbate 60 and/or vinylpyrrolidone/hexadecene copolymer.

An amount of glycerol of at least 2.5 wt %, based on the total weight of the preparation, is particularly advantageous in accordance with the invention.

It is advantageous in accordance with the invention if the preparation comprises one or more alkanediols from the group 1,2-pentanediol, 1,2-hexanediol, 1,2-octanediol, 1,2-decanediol, 2-methyl-1,3-propanediol.

It is advantageous in accordance with the invention if the preparation of the invention comprises ethanol, phenoxyethanol and/or ethylhexylglycerin.

Advantageously in accordance with the invention, the preparation of the invention comprises film formers. Film formers in the sense of the present invention are substances of various constitutions, and are characterized by the following properties: When a film former is dissolved in water or other suitable solvents, and when the solution is then applied to the skin, the film former, following evaporation of the solvent, forms a film which serves essentially to fix the photofilters on the skin and to so increase the water resistance of the product.

It is especially advantageous to select the film formers from the group of the polymers based on polyvinylpyrrolidone (PVP)

Particular preference is given to copolymers of vinylpyrrolidone, as for example the PVP hexadecene copolymer and the PVP eicosene copolymer, which are available under the trade names Antaron V216 and Antaron V220 from GAF Chemicals Corporation.

Likewise advantageous are further polymeric film formers, such as, for example, sodium polystyrene sulfonate, which is available under the trade name Flexan 130 from National Starch and Chemical Corp., and/or polyisobutene, available from Rewo under the trade name Rewopal PIB1000. Examples of further suitable polymers are polyacrylamides (Seppigel 305), polyvinyl alcohols, PVP, PVP/VA copolymers, polyglycols, acrylate/octylacrylamide copolymer (Dermacryl 79) Likewise advantageous is the use of hydrogenated castor oil dimer dilinoleate (CAS 646054-62-8, INCI Hydrogenated Castor Oil Dimer Dilinoleate), which can be acquired from Kokyu Alcohol Kogyo under the name Risocast DA-H, or else PPG-3 benzyl ether myristate (CAS 403517-45-3), which can be acquired under trade name Crodamol STS from Croda Chemicals.

In accordance with the invention is the use of the preparation of the invention for protection from skin aging (especially for protection from UV-induced skin aging) and also as a sun protection composition.

In accordance with the invention is also the use of the UV filter combination of the invention for reducing sand adhesion in cosmetic preparations (especially sunscreen compositions). In accordance with the invention in particular in this case is the combined use of microfine zinc oxide, 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 2-phenylbenzimidazole-5-sulfonic acid and/or one or more salts thereof and 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone) for reducing the sand adhesion in cosmetic preparations (especially sunscreen compositions). In this context it is particularly advantageous if the zinc oxide has a mean particle size Dn50 as determined by Laser diffraction selected in the range from 50 to 200 nm, preferably in the range from 75 to 150 nm, most preferably in the range from 90 to 130 nm and a surface coating selected from the group consisting of triethoxycaprylylsilane, octyltrimethoxysilane and dimethicone, preferably triethoxycaprylylsilane and dimethicone, most preferably triethoxycaprylylsilane.

The following examples are provided to further illustrate the compositions and effects of the present invention. These examples are illustrative only and are not intended to limit the scope of the invention in any way.

### Example 1

The formulations (O/W emulsions) as outlined in table 1 have been prepared according to standard methods in the art. Then the sand repellence has been tested according to the method as outlined below:
- Apply 30mg of cream on PMMA plate (2mg/cm²) and distribute homogenously
- Let it dry for 15 minutes
- Weigh the PMMA plate
- Put 30g of sand (Spielsand (Hornbach) Körnung von 0.06 bis 1 mm) on top of the PMMA plate such as that the surface is completely covered
- Let it dry for 15 minutes
- Turn the PMMA plate 180° without shaking
- Weigh the PMMA plate
- Calculate the amount of sand sticking on the plate
- Repeat test with 4 plates per sample

The results are outlined in Table 1

**Table 1**

| **Tradename** | **INCI** | **Ref 1** | **Inv 1** |
|---|---|---|---|
| | | **Wt.-%** | |
| Cetiol^{®} CC | Dicaprylyl carbonate | 10.00 | 10.00 |
| Uvasorb^{®} HEB | Diethylhexylbutamido triazone | 2.00 | 2.00 |
| Finsolv^{®} TN | C₁₂₋₁₅ Alkyl Benzoate | 8.00 | 8.00 |
| PARSOL^{®} Shield | Bis-ethylhexylphenol methoxyphenyl triazine | 2.00 | 2.00 |
| Dispersun DSP-OL300 | Polyhydroxystearic acid | 0.50 | 0.50 |
| Myritol^{®} 318 | Caprylic/Capric triglyceride | 10.00 | - |
| Parsol^{®} ZX | Zinc Oxide and triethoxycaprilylsilane | - | 10.00 |
| Keltrol^{®} CG-T | Xanthan Gum | 0.20 | 0.20 |
| Lanette^{®} O | Cetearyl alcohol | 1.50 | 1.50 |
| Lanette^{®} 22 | Behenyl alcohol | 1.50 | 1.50 |
| Euxyl^{®} PE 9010 | Phenoxyethanol, Ethylhexylglycerin | 1.00 | 1.00 |
| Emulgade^{®} A 6 | Ceteareth-6; stearyl alcohol | 2.00 | 2.00 |
| Eumulgin^{®} B 25 | Ceteareth-25 | 2.00 | 2.00 |
| Edeta BD | Disodium EDTA; Aqua | 0.20 | 0.20 |
| PARSOL^{®} HS | Phenylbenzimidazol sulfonic acid | 2.00 | 2.00 |
| Natriumhydroxid 30% soln. | Aqua; sodium hydroxide | 1.00 | 1.00 |
| WATER DEM. | Aqua | Ad 100 | Ad 100 |
| Glycerin 1,23 (86.5%) Ph. Eur. | Glycerin | 3.00 | 3.00 |
| Simulgel NS | Hydroxyethyl acrylate/ sodium acryloyldimethyl Taurate copolymer; Squalane, Polysorbate 60 | 0.5 | 0.5 |
| Remaining amount of sand on the plate [mg] | | 945 | 777 |
| Standard deviation [mg] | | 70 | 79 |
| % reduction (less sand) vs. Ref 1 | | | -18% |
| SPF | | 20 | 31 |

As can be retrieved from table 1, the combination according to the present invention results in a synergistic reduction of sand sticking to the plates, i.e. a synergistically improved sand repellence.

### Example 2

The formulations (O/W emulsions) as outlined in table 2 have been prepared according to standard methods in the art. Then the sand repellence has been tested according to the method as outlined below:
- Apply 2mg/ cm² of cream on PMMA plate and distribute homogenously
- Let it dry for 15 minutes @ 40°C
- Weigh the PMMA plate
- Put sand (Kultpfötchen 5kg - Terrarium Sand Natur beige | Körnung 0.4-0.8mm) into a petri dish, place the plate with cream film side on sand put a weight (500g) on plate, wait 5 minutes
- Take the plate out and turn the PMMA plate 180° without shaking
- Weigh the PMMA plate
- Calculate the amount of sand sticking on the plate
- Repeat test with 4 plates per sample

The results are outlined in Table 2

| **Tradename** | **INCI** | **#8215** | **#8213** | **#821** |
|---|---|---|---|---|
| | | **Wt.-%** | | |
| Cetiol^{®} CC | Dicaprylyl carbonate | 10.00 | 10.00 | 10.00 |
| Uvasorb^{®} HEB | Diethylhexylbutamido triazone | 2.00 | 2.00 | 2.00 |
| Finsolv^{®} TN | C12-15 Alkyl Benzoate | 8.00 | 8.00 | 8.00 |
| PARSOL^{®} Shield | Bis-ethylhexylphenol methoxyphenyl triazine | 2.00 | 2.00 | 2.00 |
| Dispersun DSP-OL300 | Polyhydroxystearic acid | 0.50 | 0.50 | 0.50 |
| ZnO-C-DS4 | Zinc oxide and dimethicone | - | 10.00 | - |
| Parsol^{®} ZX | Zinc Oxide and triethoxycaprylylsilane | - | - | 10.00 |
| Keltrol^{®} CG-T | Xanthan Gum | 0.20 | 0.20 | 0.20 |
| Lanette^{®} O | Cetearyl alcohol | 1.50 | 1.50 | 1.50 |
| Lanette^{®} 22 | Behenyl alcohol | 1.50 | 1.50 | 1.50 |
| Euxyl^{®} PE 9010 | Phenoxyethanol, Ethylhexylglycerin | 1.00 | 1.00 | 1.00 |
| Emulgade^{®} A 6 | Ceteareth-6; stearyl alcohol | 2.00 | 2.00 | 2.00 |
| Eumulgin^{®} B 25 | Ceteareth-25 | 2.00 | 2.00 | 2.00 |
| Edeta BD | Disodium EDTA; Aqua | 0.20 | 0.20 | 0.20 |
| PARSOL^{®} HS | Phenylbenzimidazol sulfonic acid | 2.00 | 2.00 | 2.00 |
| Natriumhydroxid 30% soln | Aqua; sodium hydroxide | 1.00 | 1.00 | 1.00 |
| WATER DEM. | Aqua | Ad 100 | Ad 100 | Ad 100 |
| Glycerin 1,23 (86.5%) Ph. Eur. | Glycerin | 3.00 | 3.00 | 3.00 |
| Simulgel NS | Hydroxyethyl acrylate/ sodium acryloyldimethyl Taurate copolymer; Squalane, Polysorbate 60 | 0.5 | 0.5 | 0.5 |
| Remaining amount of sand on the plate [mg] | | 768 | 682 | 524 |
| Standard deviation [mg] | | 71 | 53 | 75 |
| % reduction (less sand) vs. 8215 | | | -11% | -32% |
| % reduction (less sand) vs. 8213 | | | | -23% |

As can be retrieved from table 2, the combination according to the present invention results in a reduction of sand sticking to the plates, while the use triethoxycaprylylsilane coated zinc oxide is advantageous over the use of dimethicone coated zinc oxide.

## Claims

1. A topical composition, wherein the composition comprises a UV filter combination of
a.) a microfine zinc oxide, wherein the microfine zinc oxide has a mean particle size Dₙ50 as determined by Laser diffraction selected in the range from 30 to 200 nm and a surface coating selected from the group consisting of aluminum stearate, stearic acid, methicone, simethicone, triethoxycaprylylsilane and octyltrimethoxysilane,
b.) 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine),
c.) 2-phenylbenzimidazole-5-sulfonic acid and/or one or more salts thereof and
d.) 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone).

2. The topical composition according to anyone of the preceding claims, wherein the mean particle size Dₙ50 is selected in the range from 50 to 200 nm, preferably in the range from 75 to 150 nm, most preferably in the range from 90 to 130 nm as determined by Laser diffraction.

3. The topical composition according to any one of the preceding claims, wherein the microfine zinc oxide is surface coated with triethoxycaprylylsilane.

4. The topical composition according to any one of the preceding claims, wherein the amount of the microfine zinc oxide is selected in the range from 0.1 wt.-% to 20 wt.-%, preferably in the range from 0.5 wt.-% to 10 wt.-%, most preferably in the range of 5 wt.-% to 10 wt.-%, based on the total weight of the composition.

5. The topical composition according to any one of the preceding claims, wherein the composition contains no 3-(4-methylbenzylidene)camphor, 2-hydroxy-4-methoxybenzophenone (INCI: Oxybenzone), and ethylhexyl 2-cyano-3,3-diphenylacrylate (INCI: Octocrylene).

6. The topical composition according to any one of the preceding claims, wherein the composition is an O/W emulsion.

7. The topical composition according to any one of the preceding claims, wherein the composition comprises one or more of Ceteareth-6 and Ceteareth-25 as emulsifier.

8. The topical composition according to any one of the preceding claims, wherein the composition comprises one or more of dibutyl adipate, dicaprylyl carbonate and C12-C15 alkylbenzoate.

9. The topical composition according to any one of the preceding claims, wherein the composition comprises one or more of ethanol, p-hydroxyacetophenone, phenoxyethanol and ethylhexylglycerin.

10. The topical composition according to any one of the preceding claims, wherein the composition comprises one or more of xanthan gum, crosslinked acrylate/C10-C30 alkyl acrylate polymer, Hydroxyethyl Acrylate/Sodium Acryloyldimethyl Taurate Copolymer & Squalane & Polysorbate 60 and vinylpyrrolidone/hexadecene copolymer.

11. The topical composition according to any one of the preceding claims, wherein the composition comprises one or more of cetyl alcohol, cetearyl alcohol, stearyl alcohol and glyceryl stearate.

12. The composition according to anyone of the preceding claims, wherein the composition has an SPF of at least 20.

13. Use of microfine zinc oxide to reduce the stickiness of a topical composition comprising a 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 2-phenylbenzimidazole-5-sulfonic acid and/or one or more salts thereof and 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone).

14. Method for reducing the stickiness and/ or the sand adherence on skin of a topical composition comprising 2,4-bis{[4-(2-ethylhexyloxy)-2-hydroxy]phenyl}-6-(4-methoxyphenyl)-1,3,5-triazine (INCI: Bis-Ethylhexyloxyphenol methoxyphenyl Triazine), 2-phenylbenzimidazole-5-sulfonic acid and/or one or more salts thereof and 4,4'-((6((4 (((1,1-dimethylethyl)amino)carbonyl)phenyl)-amino)-1,3,5-triazine-2,4-diyl)diimino)bis(2-ethylhexyl)benzoate (INCI: Diethylhexyl butamido triazone) said method encompassing the step of incorporating microfine zinc oxide into said topical composition before applying said composition to skin.
